# EUROPÄISCHE PATENTSCHRIFT

(11) **EP 1 596 721 B1**
(45) Veröffentlichungstag und Bekanntmachung des Hinweises auf die Patenterteilung: **08.08.2007**
(21) Anmeldenummer: 04713066.1
(22) Anmeldetag: 20.02.2004
(51) Int. Cl.: A61B 10/00

(54) **AUFNAHMEHILFE FÜR WC-TOILETTENBECKEN**
RECEIVING DEVICE FOR A TOILET BOWL
DISPOSITIF DE RECEPTION POUR CUVETTE DE W.-C.

(30) Priorität: 25.02.2003 DE 20303129 U; 28.10.2003 DE 20316617 U
(43) Veröffentlichungstag der Anmeldung: 23.11.2005
(73) Patentinhaber: Rathenberg, Jürgen, 37581 Bad Gandersheim (DE)
(72) Erfinder: Rathenberg, Jürgen, 37581 Bad Gandersheim (DE)
(74) Vertreter: Skora, Michael
(86) Internationale Anmeldenummer: PCT/EP2004/001712
(87) Internationale Veröffentlichungsnummer: WO 2004/075757

(56) Entgegenhaltungen:
- DE-A- 3 905 572
- US-A- 2 840 826
- US-A- 5 337 426
- US-A- 5 463 782

## Beschreibung

Die Erfindung betrifft eine Aufnahmehilfe für WC-Toilettenbecken zur Aufnahme von Stuhlproben.

Bei medizinischen Untersuchungen, insbesondere bei der Darmkrebsvorsorge und bei Stoffwechsel- sowie infektiösen Magen- und Darmerkrankungen, ist es heute erforderlich, Stuhlproben zu untersuchen, die der Patient im häuslichen Bereich bei der Benutzung des Toilettenbeckens entnehmen muß. Diese Stuhlproben müssen für ein einwandfreies Untersuchungsergebnis unter hygienischen Bedingungen entnommen werden.

Es hat sich gezeigt, daß es für ein einwandfreies Untersuchungsergebnis insbesondere erforderlich ist, einen Kontakt sowohl zwischen Stuhlprobe und Spülwasser als auch zwischen Stuhlprobe und Urin möglichst zu vermeiden. Dies bereitet jedoch bei modernen Sanitäranlagen, insbesondere bei sogenannten Tiefspülbecken, Schwierigkeiten, da ein Kontakt zwischen Spülwasser, Urin und Stuhlprobe nicht vermieden wird. Ein solcher Kontakt verfälscht jedoch das Untersuchungsergebnis oder macht die Stuhlprobe für die Untersuchung unbrauchbar. Der Benutzer bzw. Patient hat also größte Schwierigkeiten, unter hygienischen Bedingungen eine Stuhlprobe zu entnehmen.

Aus DE 87 03 290 U1 ist eine Aufnahmehilfe bekannt, die aufwendig zu fertigen, kompliziert zu handhaben und schwierig zu entsorgen ist.

Aus US-A-5 463 782, von der der Oberbegriff des Anspruch 1 ausgeht, ist eine Falt-Stuhlproben-Aufnahmehilfe bekannt, welche zur Befestigung an einem WC-Toilettenbecken drei Laschen aufweist, auf denen Befestigungsmittel angeordnet sind. Mit den Befestigungsmitteln wird die Aufnahmehilfe an einer Toilettenbrille befestigt. Die Aufnahmehilfe ist derart vorgefaltet, daß beim Auseinanderfalten ein Stuhlproben-Aufnahmebereich entsteht, welcher einem Quaderausschnitt entspricht. Diese Aufnahmehilfe ist mit einem erheblichen Fertigungs- und Materialaufwand verbunden und daher teuer.

Aus US-A-5 337 426 ist eine rechteckige Aufnahmehilfe aus Papier bzw. Zellstoff bekannt, welche lange, sichelförmige Schlitze aufweist. Zum Gebrauch wird die Aufnahmehilfe von vorne auf eine Toilettenbrille aufgeschoben. Durch das Eigengewicht hängt die Aufnahmehilfe in der Mitte durch und bildet eine Mulde. Auch diese Aufnahmehilfe erfordert viel Material und ist daher ebenfalls teuer.

Aus DE 92 17 885 U1 ist eine Aufnahmehilfe bekannt, die nur aus einem Aufnahmeblatt besteht, einfach zu fertigen und einfach zu handhaben ist. Diese Aufnahmehilfe ist biologisch abbaubar und daher ökologisch unbedenklich. Sie basiert auf einem langgestreckten Aufnahmeblatt, das im Bereich der beiden sich in Längserstreckung gegenüberliegenden Enden jeweils einen Befestigungsbereich mit klebender Wirkung aufweist und mit diesen im Bereich des Beckenrandes eines WC-Toilettenbeckens befestigt wird.

Die klebende Wirkung der Befestigungsbereiche wird durch Aufsprühen eines Schmelzhaftklebers erzielt.

Um ein Verschmutzen der Befestigungsbereiche vor der Verwendung der Aufnahmehilfe zu verhindern, sind diese zu Transport- und Lagerzwecken durch Gegenlagen abgedeckt, die am Aufnahmeblatt selbst ausgebildet und durch Falzung des Aufnahmeblattes erzielt sind. Der vor dem Falzen durch Sprühtechnik zur Bildung der Befestigungsbereiche aufgetragene Schmelzhaftkleber kann jedoch bereits kurze Zeit nach dem Falten zum Festkleben an der Gegenlage neigen und kann die Aufnahmehilfe dadurch unbrauchbar machen.

Aus US-A-2 840 826 ist eine Aufnahmehilfe für WC-Toilettenbecken mit einem langgestreckten Aufnahmeblatt bekannt, wobei dessen Länge ein Mehrfaches der Breite beträgt. An den Außenseiten des Aufnahmeblattes sind Klebebereiche vorgesehen, mit denen das Aufnahmeblatt an einer Toilettenbrille befestigt werden kann. Das Blatt hängt im Gebrauchszustand im Toilettenbecken herunter.

Es besteht das Problem, daß infektiöser Stuhlgang häufig dünnflüssig ist, von dem Aufnahmeblatt herabfließt und so eine Stuhlprobenentnahme von dem Aufnahmeblatt in diesem Fall nicht möglich ist.

Auf diesem Hintergrund liegt der Erfindung die Aufgabe zugrunde, eine Aufnahmehilfe zur Verfügung zu stellen, mit der Stuhlproben unabhängig von deren Konsistenz sicher gewonnen werden können, und die ferner kostengünstig zu fertigen ist.

Die Lösung der Aufgabe erfolgt erfindungsgemäß mit den Merkmalen des Anspruchs 1.

Gemäß der Erfindung ist bei einer Aufnahmehilfe mit einem langgestreckten Aufnahmeblatt vorgesehen, daß durch das Aufnahmeblatt im Gebrauchszustand ein eine Mulde beschreibender Stuhlproben-Aufnahmebereich zur Verfügung gestellt wird. Durch die Ausbildung eines solchen muldenförmigen Stuhlproben-Aufnahmebereiches kann sichergestellt werden, daß nicht nur fester Stuhlgang, sondern auch infektiöser, dünnflüssiger Stuhlgang nicht von dem Aufnahmeblatt herunterfließt und dadurch - in dem muldenartigen Stuhlproben-Aufnahmebereich gesammelt - für die Entnahme einer Stuhlprobe ohne vorherigen Kontakt mit Spülwasser oder Urin zur Verfügung steht. Bemerkenswert ist dabei, daß die Aufnahmehilfe mit ihrem muldenartigen Aufnahmebereich im Gebrauchszustand eine räumliche Struktur darstellt, die gleichwohl in der Herstellung, Lagerung und im Versand flach ist und sich vor der Verwendung nur in der Ebene erstreckt.

Der eine Mulde beschreibende Stuhlproben-Aufnahmebereich durch eine Verwerfung des Aufnahmeblattes gebildet. Dadurch kann durch ein einfaches Auseinanderfalten des im Lagerzustand flachen Aufnahmeblattes an diesem selbst eine räumliche Struktur ausgebildet werden, ohne daß dazu zusätzliche Elemente, wie Einsätze o.ä. erforderlich sind. Dadurch kann ferner an einer derart ausgestalteten Aufnahmehilfe die durch die Erfindung möglich gewordene Funktionalitätssteigerung praktisch ohne Materialaufwand und ohne Mehrgewicht gegenüber den Aufnahmehilfen des Standes der Technik realisiert werden.

Die Mulde weist Ränder auf, die durch die Längskanten des Aufnahmeblattes gebildet sind. Dadurch steht praktisch die gesamte Aufnahmehilfe als Gefäß zur Verfügung.

Die Verwerfung des Aufnahmeblattes wird durch eine Verkürzung der wirksamen Länge der Längskanten des Aufnahmeblattes erreicht. Dies wird dadurch realisiert, daß an dem Aufnahmeblatt mindestens an dessen Seitenrand an einem ersten Bereich und mindestens an einem, dem ersten Bereich bezüglich der Längsachse des Aufnahmeblattes gegenüberliegenden, zweiten Bereich eine Faltung vorgenommen wird und diese Faltung, die im Gebrauchszustand allerdings nur im Randbereich wirksam ist, durch eine Formfixierung auch bei Belastung bestehen bleibt. Dadurch kann ein Benutzer das Aufnahmeblatt vor dem Gebrauch einfach auseinanderfalten und mit dessen Befestigungsbereichen an einem Beckenrand eines WC-Toilettenbeckens befestigen, wobei sich aufgrund der verschiedenen wirksamen Längen am Rand und in der Mitte des Aufnahmeblattes eine Vertiefung in der Mitte des Aufnahmeblattes ausbildet.

Die belastungsfähige Formfixierung kann in einer bevorzugten Ausführungsform durch eine flache Niet-, Klemm- oder Steckverbindung erfolgen. Wenn für die Nietverbindung mehrere, alternativ verwendbare Löcher in dem Aufnahmeblatt vorgesehen sind, kann - sofern die Fixierung durch den Benutzer erfolgt - eine Einstellung der Form und Größe des Stuhlproben-Aufnahmebereiches durch den Benutzer erfolgen. Bei Klemm- oder Steckverbindungen, insbesondere bei einer Fixierung der Faltung in den Randbereichen durch eine Heftklammer o.ä., ist auch eine Vorjustierung durch den Hersteller möglich, und bei Bedarf kann die Form bzw. Größe des Stuhlproben-Aufnahmebereiches durch einfaches Verschieben der Heftklammer nachjustiert werden. Zur Vergrößerung des Stuhlproben-Aufnahmebereiches wird die Heftklammer dann so verschoben, daß die durch die Faltung und Formfixierung der Seitenränder aneinander liegenden Flächen sich vergrößern und damit die Randlängen verkürzt werden.

Gemäß einer anderen bevorzugten Ausführungsform ist eine Klebverbindung als belastungsfähige Formfixierung der Faltung vorgesehen. Zwar kann mit der Klebverbindung keine individuelle Anpassung des Stuhlproben-Aufnahmebereiches mehr erfolgen, jedoch läßt sich eine solche Aufnahmehilfe zum einen besonders kostengünstig und einfach herstellen, zum anderen sind Fehleinstellungen des Benutzers ausgeschlossen, da die Aufnahmehilfe vom Benutzer lediglich auseinandergefaltet und - ohne jegliche Einstellungen - an einem Beckenrand eines WC-Toilettenbeckens befestigt werden kann.

Bei einer weiter bevorzugten Ausführungsform einer erfindungsgemäßen Aufnahmehilfe ist der eine Mulde beschreibende Stuhlproben-Aufnahmebereich durch eine Vertiefungs-Prägung des Aufnahmeblattes gebildet. Dann kann das Aufnahmeblatt einstückig ausgebildet werden, was insbesondere bei einer Massenfertigung niedrige Herstellungskosten bewirkt.

Die Aufnahmehilfe ist in einer weiter bevorzugten Ausführungsform aus einem in Wasser langsam zerfallenden Material, insbesondere Papier, hergestellt und ist daher vollständig biologisch abbaubar.

Somit kann die Aufnahmehilfe nach dem Gebrauch durch die Kanalisation entsorgt werden, ohne Gefahr zu laufen, die Umwelt zu verschmutzen oder die Kanalisation langfristig zu verstopfen.

Ein Schmelzhaftkleber, der nur auf die Befestigungsbereiche aufgesprüht ist, ist gemäß einer bevorzugten Ausführung ebenfalls biologisch abbaubar und daher ökologisch unbedenklich. Durch das Verfahren des Aufsprühens ist eine besonders dünne Beschichtung des Papiers möglich, wodurch einerseits Klebstoff bei der Herstellung eingespart wird und andererseits dünnes Papier zur Herstellung verwendet werden kann, welches ebenfalls kostengünstiger ist als dickeres Spezialpapier, welches bei anderen Beschichtungsmethoden verwendet werden müßte.

In einer weiter bevorzugten Ausführung einer erfindungsgemäßen Aufnahmehilfe sind der erste Befestigungsbereich und der mindestens zweite Befestigungsbereich zu Transport und/oder Lagerzwecken durch eine erste Gegenlage und eine mindestens zweite Gegenlage abgedeckt. Somit wird verhindert, daß die Befestigungsbereiche schon vor dem Gebrauch der Aufnahmehilfe ihre Klebwirkung verlieren und somit unbrauchbar werden.

Um während des Transportes und während der Lagerung von Aufnahmehilfen das Verkleben von Befestigungsbereich und Gegenlage zu verhindern, ist bei einer weiter bevorzugten Ausführung der Erfindung eine klebstoffabweisende Substanz auf die Gegenlage aufgetragen.

Wenn die auf der Gegenlage aufgetragene klebstoffabweisende Substanz Dissacharide, insbesondere Maltose und Glukose, sowie Polysacharide, Lactose und längerkettige Alkohole aufweist und auf wäßriger Basis beruht, wird ein Verkleben der Befestigungsbereiche an den Gegenlagen verhindert. Daher kann eine derart ausgestaltete Aufnahmehilfe bedeutend länger gelagert werden.

Alternativ zu der oben beschriebenen klebstoffabweisenden Substanz oder zusätzlich dazu kann eine Verringerung der Klebwirkung zwischen Befestigungsbereich und Gegenlage dadurch erreicht werden, daß das Material des Aufnahmeblattes in dem Anlagebereich zwischen Befestigungsbereich und Gegenlage nicht vollflächig zur Anlage gebracht wird. Dies geschieht vorzugsweise durch eine Prägung des Papiers, die zu einer Ausbildung von erhabenen bzw. vertieften Zonen führt.

Die Fertigung wird erheblich vereinfacht, wenn das gesamte Aufnahmeblatt mit Ausnahme des Befestigungsbereiches geprägt wird, da dann auf bekannte Herstellungsverfahren von handelsüblichem Papier aus der Massenfertigung zurückgegriffen werden kann und weniger Zeit für die Werkzeugrüstung benötigt wird.

Weitere vorteilhafte Ausgestaltungen und Weiterbildungen der Erfindung ergeben sich aus den Unteransprüchen sowie aus der Beschreibung im Zusammenhang mit den Zeichnungen.

Es zeigen:
- Fig. 1: eine bevorzugte Ausführungsform einer erfindungsgemäßen Aufnahmehilfe für WC-Toilettenbecken im Verwendungszustand in einer perspektivischen Ansicht,
- Fig. 2: die Aufnahmehilfe in Fig. 1 in einer Ansicht gemäß dem eingezeichneten Schnitt II-II in Fig. 1,
- Fig. 3: die Oberseite der Aufnahmehilfe in den Fig. 1 und 2 mit Klebstellen und Verwerfungen in einem ausgefalteten Zustand,
- Fig. 4: die Aufnahmehilfe in den Fig. 1 bis 3 in einem ausgefalteten Zustand in einer Seitenansicht,
- Fig. 5: die Unterseite der Aufnahmehilfe in den Fig. 1 bis Fig. 4 in einem ausgefalteten Zustand mit Verwerfungen, Befestigungsbereichen und Gegenlagen,
- Fig. 6: die Aufnahmehilfe in den Fig. 1 bis Fig. 5 mit Befestigungsbereichen, Gegenlagen und Klebschichten in einem teilweise zusammengefalteten Zustand in einer Seitenansicht,
- Fig. 7: eine Detailansicht Y der Aufnahmehilfe in den Fig. 1 bis 6 gemäß dem eingezeichneten Schnitt VIII-VIII in Fig. 3 und gemäß der Markierung in Fig. 6,
- Fig. 8: eine Detailansicht Z der Aufnahmehilfe in den Fig. 1 bis 6 gemäß der Markierung in Fig. 6.

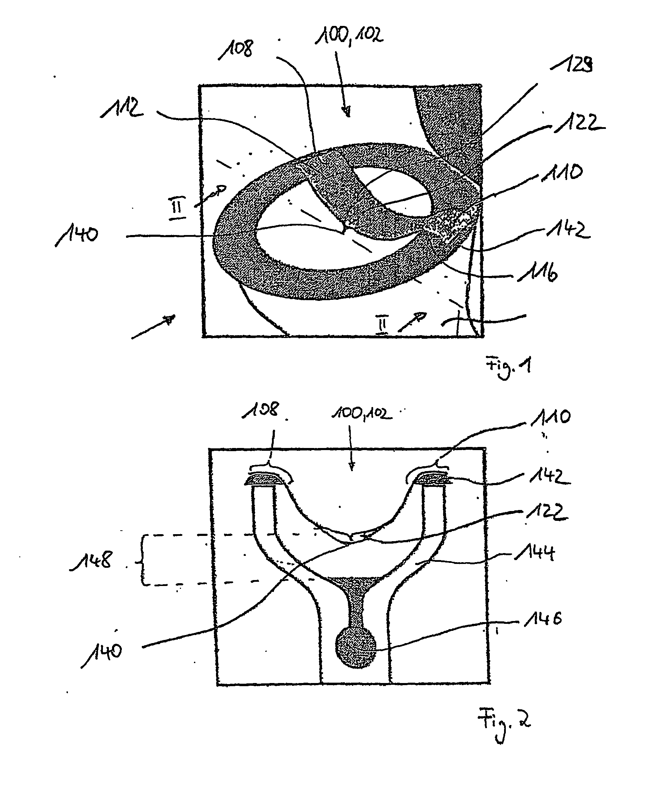

Die in den Fig. 1 bis 8 gezeigte Aufnahmehilfe 100 besteht im wesentlichen aus einem Aufnahmeblatt 102 mit einer Länge von 40 bis 80 cm, vorzugsweise 48 cm. Die Breite beträgt zwischen 10 cm und 25 cm, vorzugsweise 15 cm.

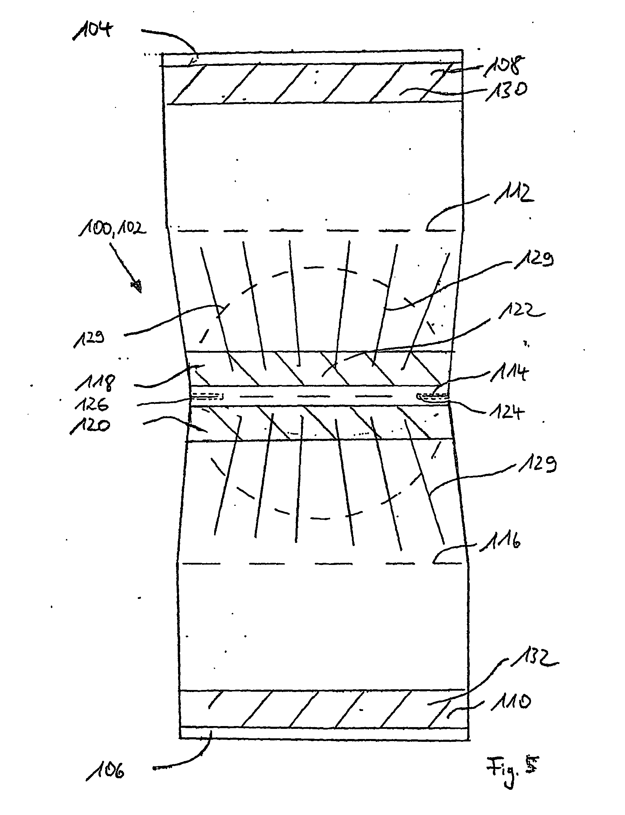

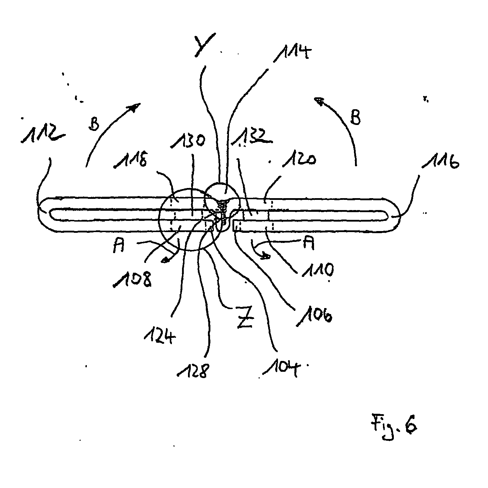

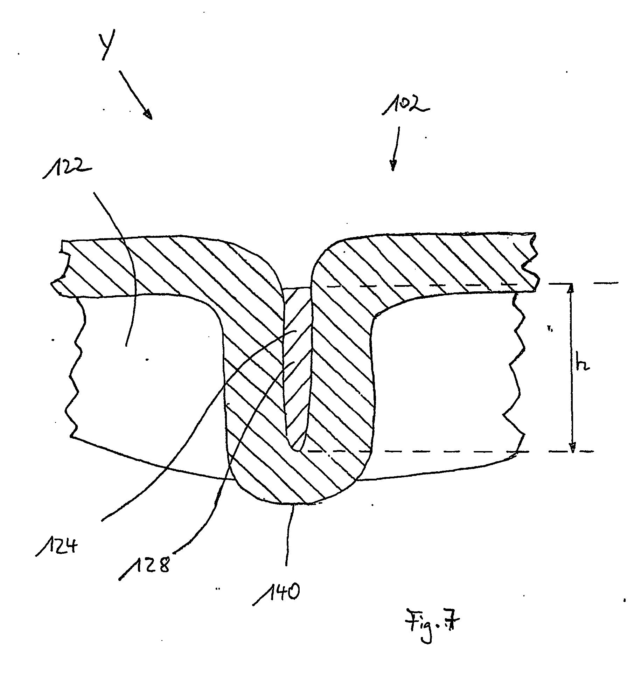

Wie in Fig. 5 zu erkennen ist, befinden sich an dem ersten Ende 104 und an dem zweiten Ende 106 der langgestreckten Seiten ein erster Befestigungsbereich 108 und ein zweiter Befestigungsbereich 110, wobei die Breite des ersten Befestigungsbereiches 108 und des zweiten Befestigungsbereiches 110 zwischen 1 und 3 cm beträgt, vorzugsweise 2 cm. Gestrichelt eingezeichnet sind eine erste Falzstelle 112, eine zweite Falzstelle 114 und eine dritte Falzstelle 116, an denen das Aufnahmeblatt 102 zum Transport bzw. zur Lagerung gefalzt wird. Im Bereich der Mitte der langgestreckten Seiten befinden sich parallel zu der als Falzlinie ausgebildeten zweiten Falzstelle 114 zwei Bereiche, die im gefalteten Zustand des Aufnahmeblattes 102 als erste Gegenlage 118 und als zweite Gegenlage 120 des ersten Befestigungsbereiches 108 bzw. zweiten Befestigungsbereiches 120 fungieren.

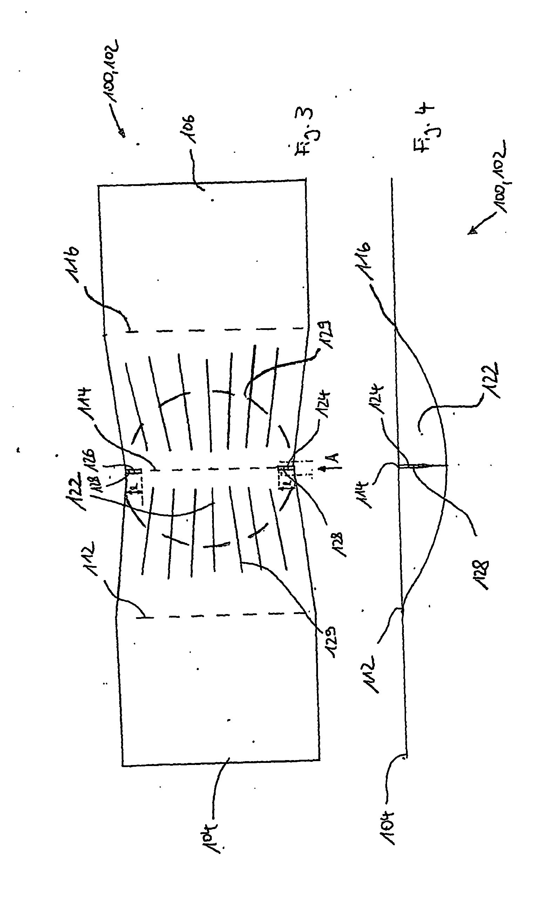

Wie in Fig. 3 gezeigt, weist die Oberseite des Aufnahmeblattes 102 im Bereich der zweiten Falzstelle 114 (Falzlinie) einen Stuhlproben-Aufnahmebereich 122 auf. Dieser ist wie nachfolgend beschrieben durch eine Verwerfung des Papiers gebildet.

Zur Herstellung der Aufnahmehilfe 100 wird in einem ersten Klebebereich 124 und einem zweiten Klebebereich 126 jeweils im Randbereich der Längskanten des Aufnahmeblattes 102 Klebstoff 128 appliziert. Der Klebstoff 128 kann jeweils als Klebepunkt appliziert werden. Nach Applizierung des Klebstoffs 128 wird das Aufnahmeblatt entlang der Falzlinie 114 gefaltet. Dies führt in den Klebebereichen 124, 126 zu einer Klebverbindung. Das Aufnahmeblatt 102 erfährt so an seinen Längskanten in den Klebebereichen 124, 126 eine belastungsfähige Formfixierung. Die Höhe h der Klebverbindung kann im ersten und zweiten Klebebereich 124, 126 variiert werden. Das Längenmaß 1 des ersten bzw. zweiten Klebebereiches 124, 126, gemessen jeweils von der Längskante des Aufnahmeblattes 102 entlang der Falzlinie 114, beträgt bei einer Klebverbindung mit Klebebereichen 124, 126 5 bis 20 mm, vorzugsweise 10 mm.

Wie in Fig. 3 zu erkennen, führt die Klebverbindung zu einer Verkürzung der wirksamen Länge der Längskanten des Aufnahmeblattes 102. Durch die Verkürzung der wirksamen Länge der Längskanten des Aufnahmeblattes bilden sich Verwerfungen 129 aus. In dem Bereich zwischen dem ersten und zweiten Klebebereich 124, 126 hängt das Aufnahmeblattes 102 durch,' wodurch ein muldenartiger Stuhlproben-Aufnahmebereich 122 gebildet wird. Als Klebstoff 128 ist insbesondere ein Hot-Melt-Kleber vorgesehen, mit welchem eine permanente Verklebung erzielt wird. Zur Ausbildung des muldenartigen Stuhlproben-Aufnahmebereiches 122 muß das Aufnahmeblatt 102 bei dieser Ausführung von einem Benutzer lediglich an einem Beckenrand eines WC-Toilettenbeckens befestigt werden. Die Ausbildung des muldenartigen Stuhlproben-Aufnahmebereiches 122 erfolgt dann selbsttätig durch herabfallenden Stuhl. Die Ausbildung kann alternativ auch vorher von Hand durch Ausstreichen erfolgen.

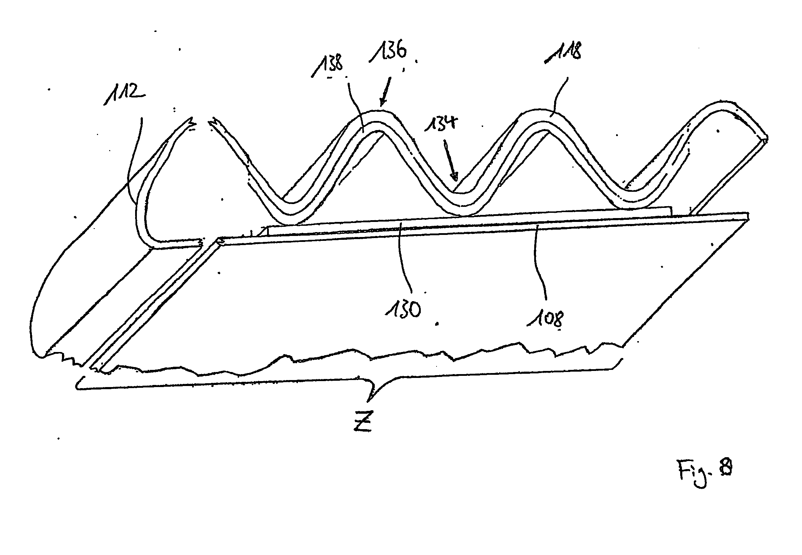

Wie in den Fig. 3 und 5 zu sehen, führt die Verkürzung der wirksamen Länge der Längskanten des Aufnahmeblattes 102 ferner im ausgefalteten Zustand zu einer Verjüngung des Aufnahmeblattes 102 zwischen der ersten Falzstelle 112 und der dritten Falzstelle 116 in dessen Randbereichen. Der Zusammenhang zwischen dem ersten bzw. zweiten Klebebereich 124, 126 und der Verjüngung wird anhand von Fig. 7 näher erläutert.

Fig. 5 und 6 zeigen, wie auf dem im wesentlichen aus glattem Papier bestehenden ersten bzw. zweiten Befestigungsbereich 108, 110 eine erste Klebschicht 130 bzw. eine zweite Klebschicht 132 aufgebracht sind, um die klebende Wirkung für die Befestigungsfunktion zu erzielen. Für die erste Klebschicht 130 und die zweite Klebschicht 132 wird insbesondere ein Adhesiv-Kleber verwendet, der nach der Verwendung der Aufnahmehilfe ein leichtes Lösen des Aufnahmeblattes von dem WC-Toilettenbecken ermöglicht.

Die Breite des ersten Befestigungsbereiches 108 und des zweiten Befestigungsbereiches 110 ist mit einer Sicherheitstoleranz beaufschlagt, so daß die erste Klebschicht 130 und die zweite Klebschicht 132 sich nicht über die gesamte Breite des ersten bzw. zweiten Befestigungsbereiches 108, 110 erstrecken. Im zusammengefalteten Zustand liegen die erste Klebschicht 130 und die zweite Klebschicht 132 des ersten Befestigungsbereiches '108 bzw. zweiten Befestigungsbereiches 110 an der ersten Gegenlage 118 bzw. zweiten Gegenlage 120 an. Die erste Gegenlage 118 und die zweite Gegenlage 120 bestehen, wie alle anderen Bereiche des Aufnahmeblattes 102 mit Ausnahme des ersten Befestigungsbereiches 108 und des zweiten Befestigungsbereiches 110, im wesentlichen aus geprägtem Papier mit erhabenen Zonen 134 und vertieften Zonen 136. Im gefalzten Zustand liegen folglich nur die erhabenen Zonen 134 der ersten bzw. der zweiten Gegenlage 118, 120 an der ersten bzw. zweiten Klebschicht 130, 132 des ersten bzw. zweiten Befestigungsbereiches 108, 110 an, wodurch sich die Klebwirkung des ersten und des zweiten Befestigungsbereiches 108, 110 an der ersten bzw. der zweiten Gegenlage 118, 120 verringert. Dies verbessert insbesondere die Lager- und Transportfähigkeit, da ein ungewolltes Verkleben wirksam vermieden wird.

Fig. 7 zeigt einen Schnitt durch das Aufnahmeblatt 102 im Randbereich des zweiten Falzbereiches 114 gemäß dem Schnitt VI-VI in Fig. 4. Gleichzeitig entspricht Fig. 6 der in Fig. 5 eingezeichneten Detailansicht Y. Es ist zu erkennen, daß das Aufnahmeblatt 102 so gefaltet ist, daß die Falzkante 140 nach unten weist. Das Aufnahmeblatt 102 wird durch Klebstoff 128 auf einer Höhe h von 2 bis 10 mm, vorzugsweise 5 mm, zusammengehalten. Dadurch bleibt das Aufnahmeblatt 102 im Bereich des ersten bzw. zweiten Klebebereichs 124, 126 sowohl im auseinandergefalteten als auch im zusammengefalteten Zustand gefalzt und ist belastungsfähig formfixiert. Je größer die Höhe h gewählt ist, desto größer ist die Verkürzung der wirksamen Länge der Längskanten des Aufnahmeblattes 102 und desto stärker fällt auch die Verjüngung der Ränder zwischen der ersten Falzstelle 112 und der dritten Falzstelle 116 aus. Je größer die Verjüngung zwischen der ersten Falzstelle 112 und der dritten Falzstelle 116 ist, desto tiefer ist der eine Mulde beschreibende Stuhlproben-Aufnahmebereich 122.Die Länge 1 des ersten bzw. des zweiten Klebebereichs 124, 126 beträgt 5 bis 20 mm, vorzugsweise 10 mm. Die Höhe h kann auch dann variiert werden, wenn die Klebebereiche punktförmig sind.

Fig. 8 zeigt am Beispiel der ersten Gegenlage 118, daß das geprägte Papier der Gegenlage 118 zusätzlich eine klebstoffabweisende Schicht 138 aufweist. Die klebstoffabweisende Schicht 138 erstreckt sich in ihrer Breitenausdehnung etwas weiter als die gegenüberliegende erste Klebschicht 130, damit sichergestellt ist, daß im gefalteten Zustand des Aufnahmeblattes 102 die erste Klebschicht 130 immer auf einen Abschnitt der ersten Gegenlage 118 trifft, der eine klebstoffabweisende Schicht 138 aufweist und somit die Haftung weiter verringert.

In den Fig. 1 und 2 ist gezeigt, wie das Aufnahmeblatt 102 mit seinem ersten bzw. zweiten Befestigungsbereich 108, 110 an einem Beckenrand 142 eines WC-Toilettenbeckens 144 befestigt ist. Die Anordnung des ersten bzw. zweiten Befestigungsbereiches 108, 110 an dem Aufnahmeblatt 102 ist so bemessen, daß das Aufnahmeblatt 102 im WC-Toilettenbecken 144 frei herunterhängt, ohne in Kontakt mit dem Toilettenwasser 146 zu kommen, wobei ein Sicherheitsabstand 148 verbleibt. Dadurch wird gewährleistet, daß kein Kontakt zwischen Stuhlprobe und Toilettenwasser 146 entsteht. Der Kontakt zwischen Urin und Stuhlprobe wird dadurch vermieden, daß das Aufnahmeblatt 102, wie in Fig. 2 zu sehen ist, im hinteren Bereich des Beckenrandes 142 des WC-Toilettenbeckens 144 angebracht ist.

Zum Versand wird das in Fig. 5 dargestellte Aufnahmeblatt 102 zusätzlich zu der ersten und dritten Falzstelle 112, 116 an der zweiten Falzstelle 114 in Richtung der Pfeile B so gefaltet, daß die erste Gegenlage 118 und die zweite Gegenlage 120 aneinander anliegen und sich eine kompakte, ebene Lagerform des Aufnahmeblattes 102 ergibt.

Um das Aufnahmeblatt 102 ausgehend von dem in Fig. 6 gezeigten Zustand auseinanderzufalten, werden das erste bzw. zweite Ende 104, 106 in Richtung der Pfeile A gezogen, so daß sich der erste bzw. zweite Befestigungsbereich 108, 110 von der ersten bzw. zweiten Gegenlage 118, 120 lösen und das erste bzw. zweite Ende 104, 106 anschließend, wie in Fig. 3 gezeigt, maximal weit voneinander entfernt sind.

## Patentansprüche

1. Aufnahmehilfe (100) für WC-Toilettenbecken (144) zur Aufnahme von Stuhlproben mit einem Aufnahmeblatt (102), wobei an dem Aufnahmeblatt (102) ein erster und ein mindestens zweiter Befestigungsbereich (108, 110) zur lösbaren Befestigung im Bereich eines Beckenrandes (142) eines WC-Toilettenbeckens (144) vorgesehen sind,
wobei durch das Aufnahmeblatt (102) im Gebrauchszustand ein eine Mulde beschreibender Stuhlproben-Aufnahmebereich (122) zur Verfügung gestellt wird,
wobei der eine Mulde beschreibende Stuhlproben-Aufnahmebereich (122) durch eine einerseits durch Faltung bewirkte Verwerfung (129) des Aufnahmeblattes (102) gebildet ist,
**dadurch gekennzeichnet,**
**daß** das Aufnahmeblatt (102) langgestreckt ist und seine Länge ein Mehrfaches der Breite beträgt,
**daß** die Mulde Ränder aufweist, die durch die Längskanten des Aufnahmeblattes (102) gebildet sind,
wobei die Verwerfung (129) des Aufnahmeblattes andererseits (102) durch eine Verkürzung der wirksamen Länge der Längskanten des Aufnahmeblattes (102) hervorgerufen ist, und
wobei die Verkürzung der wirksamen Länge der Längskanten des Aufnahmeblattes (102) durch eine Faltung und eine belastungsfähige Formfixierung der Faltung eines ersten Bereiches am Seitenrand des Aufnahmeblattes (102) und mindestens eines, dem ersten Bereich bezüglich der Längsachse des Aufnahmeblattes (102) gegenüberliegenden, zweiten Bereiches am Seitenrand des Aufnahmeblattes (102) ausgebildet ist.

2. Aufnahmehilfe (100) nach Anspruch 1, **dadurch gekennzeichnet, daß** die belastungsfähige Formfixierung eine flache Niet-, Klemm- oder Steckverbindung ist.

3. Aufnahmehilfe (100) nach Anspruch 1, **dadurch gekennzeichnet, daß** die belastungsfähige Formfixierung eine Klebverbindung ist.

4. Aufnahmehilfe (100) nach Anspruch 3, **dadurch gekennzeichnet, daß** der eine Mulde beschreibende Stuhlproben-Aufnahmebereich (122) durch eine Vertiefungs-Prägung des Aufnahmeblattes (102) an dem Aufnahmeblatt (102) selbst gebildet ist.

5. Aufnahmehilfe (100) nach einem der Ansprüche 1 bis 4, **dadurch gekennzeichnet, daß** das Aufnahmeblatt (102) aus einem in Wasser langsam zerfallenden Material, insbesondere aus Papier besteht.

6. Aufnahmehilfe (100) nach einem der Ansprüche 1 bis 5, **dadurch gekennzeichnet, daß** der erste Befestigungsbereich (108) und der mindestens zweite Befestigungsbereich (110) durch Aufsprühen eines Schmelzhaftklebers mit ökologisch unbedenklicher Basis ausgebildet sind.

7. Aufnahmehilfe (100) nach einem der Ansprüche 1 bis 6, **dadurch gekennzeichnet, daß** der erste Befestigungsbereich (108) und der mindestens zweite Befestigungsbereich (110) zu Transport- und/oder Lagerzwecken durch eine erste Gegenlage (118) und eine mindestens zweite Gegenlage (120) abgedeckt sind.

8. Aufnahmehilfe (100) nach Anspruch 7, **dadurch gekennzeichnet, daß** das Material der ersten Gegenlage (118) und der mindestens zweiten Gegenlage (120) zur Verringerung der Klebwirkung, insbesondere durch den Auftrag einer klebstoffabweisenden Substanz (138), behandelt sind.

9. Aufnahmehilfe (100) nach Anspruch 8, **dadurch gekennzeichnet, daß** die klebstoffabweisende Substanz (138) Disaccharide, insbesondere Maltose und Glucose sowie Polysaccharide, Lactose und längerkettige. Alkohole aufweist und auf wäßriger Basis beruht.

10. Aufnahmehilfe (100) nach einem der Ansprüche 7 bis 9, **dadurch gekennzeichnet, daß** die erste Gegenlage (118) und die mindestens zweite Gegenlage (120) an dem Aufnahmeblatt (102) ausgebildet sind und durch Falzung an Falzstellen (112, 114, 116) des Materials des Aufnahmeblattes (102) an dem ersten Befestigungsbereich (108) und dem mindestens zweiten Befestigungsbereich (110) zu Transport- und/oder Lagerzwecken zur Anlage gebracht sind.

11. Aufnahmehilfe (100) nach Anspruch 10, **dadurch gekennzeichnet, daß** der erste Befestigungsbereich (108) und der mindestens zweite Befestigungsbereich (110) an der ersten Gegenlage (118) bzw. an der mindestens zweiten Gegenlage (120) nicht vollflächig zur Anlage gebracht sind, wozu das Material der ersten und der mindestens zweiten Gegenlage (118, 120) durch Prägung ausgebildete erhabene bzw. vertiefte Zonen (134, 136) aufweist.

12. Aufnahmehilfe (100) nach einem der Ansprüche 1 bis 11, **dadurch gekennzeichnet, daß** das gesamte Aufnahmeblatt (102) mit Ausnahme der Befestigungsbereiche (108, 110) durch Prägung so verändert ist, daß das Material in geprägten Bereichen erhabene bzw. vertiefte Zonen (134, 136) aufweist.

## Claims

1. Receiving device (100) for toilet bowls (144) used for collecting stool samples having a collection sheet (102),
wherein a first and at least a second fixing area (108, 110) are provided on the collection sheet (102) for detachable securing to an area at the edge (142) of a toilet bowl (144),
wherein said collection sheet (102) yields a trough-defined stool sample receiving area (122) when in the state of use,
wherein the trough-defined stool sample receiving area (122) is formed on the one hand by a distortion (129) resulting from folding said collection sheet (102),
**characterized in that**,
the collection sheet (102) is elongated and its length amounts to several times its width,
the trough exhibits edges formed by the longitudinal edges of the collection sheet (102),
wherein the distortion (129) of the collection sheet (102) is effected on the other hand by shortening the effective length of the longitudinal edges of the collection sheet (102), and
wherein shortening the effective length of the longitudinal edges of the collection sheet (102) is realized by a fold and a stress-resistant form-fixing of said fold at a first area on the lateral edge of the collection sheet (102) and at least at one second area on the lateral edge of the collection sheet (102) opposite the first area relative the longitudinal axis of said collection sheet (102).

2. Receiving device (100) according to claim 1, **characterized in that** the stress-resistant form-fixing is a flat riveted, clamping or locking connection.

3. Receiving device (100) according to claim 1, **characterized in that** the stress-resistant form-fixing is an adhesive bond.

4. Receiving device (100) according to claim 3, **characterized in that** the trough-defined stool sample receiving area (122) is formed on the collection sheet (102) itself by impression-molding of said collection sheet (102).

5. Receiving device (100) according to any one of claims 1 to 4, **characterized in that** the collection sheet (102) is made of a material which decomposes slowly in water, in particular paper.

6. Receiving device (100) according to any one of claims 1 to 5, **characterized in that** the first fixing area (108) and the at least one second fixing area (110) are realized by spraying on an environmentally-safe hot-melt adhesive.

7. Receiving device (100) according to any one of claims 1 to 6, **characterized in that** the first fixing area (108) and the at least one second fixing area (110) are covered by a first backing (118) and at least one second backing (120) for transport/storage purposes.

8. Receiving device (100) according to claim 7, **characterized in that** the material of the first backing (118) and the at least one second backing (120) are treated so as to minimize the adhesive effect, in particular by application of an adhesive-repelling substance (138).

9. Receiving device (100) according to claim 8, **characterized in that** the adhesive-repelling substance (138) consists of dissaccharides, in particular maltose or glucose, or polysaccharides, lactose or long-chained alcohols and is water-based.

10. Receiving device (100) according to any one of claims 7 to 9, **characterized in that** the first backing (118) and the at least second backing (120) are formed on the collection sheet (102) and positioned against the first fixing area (108) and the at least second fixing area (110) by the folds at folded areas (112, 114, 116) of the material of the collection sheet (102) for transport/storage purposes.

11. Receiving device (100) according to claim 10, **characterized in that** the first fixing area (108) and the at least one second fixing area (110) are not positioned fully against the first backing (118), the at least one second backing (120) respectively, whereby the material of the first and the at least second backing (118, 120) exhibit raised and depressed areas (134, 136) formed by embossing.

12. Receiving device (100) according to any one of claims 1 to 11, **characterized in that** the entire collection sheet (102), with the exception of fixing areas (108, 110), is modified by embossing to the extent that the material in the embossed areas exhibits raised and depressed areas (134, 136).

## Revendications

1. Dispositif de réception (100) pour cuvette de W-C (144) pour la réception d'échantillons de selles par une feuille de réception (102), une première et au moins une deuxième zone de fixation (108, 110) pour la fixation amovible dans la zone d'un bord de cuvette (142) d'une cuvette de W-C (144) étant prévues à la feuille de réception (102),
une zone de réception d'échantillons de selles (122) constituant une cuvette étant proposée par la feuille de réception (102) en l'état d'utilisation,
la zone de réception d'échantillons de selles (122) constituant une cuvette étant formée d'une part par un rejet (129) réalisé par pliage de la feuille de réception (102),
**caractérisé en ce que**
la feuille de réception (102) est allongée et sa longueur est un multiple de la largeur,
la cuvette présente des bords formés par les arêtes longitudinales de la feuille de réception (102),
le rejet (129) de la feuille de réception (102) résultant, d'autre part, d'un raccourcissement de la longueur efficace des arêtes longitudinales de la feuille de réception (102) et
le raccourcissement de la longueur efficace des arêtes longitudinales de la feuille de réception (102) étant constitué par un pli et une mise en une forme apte à supporter une charge du pli d'une première zone au bord latéral de la feuille de réception (102) et au moins d'une deuxième zone au bord latéral de la feuille de réception (102), située en face de la première zone par rapport à l'axe longitudinal de la feuille de réception (102).

2. Dispositif de réception (100) selon la revendication 1, **caractérisé en ce que** la mise en une forme apte à supporter une charge est une liaison plate par rivetage, serrage ou enfichage.

3. Dispositif de réception (100) selon la revendication 1, **caractérisé en ce que** la mise en une forme apte à supporter une charge est une liaison par adhésif.

4. Dispositif de réception (100) selon la revendication 3, **caractérisé en ce que** la zone de réception d'échantillons de selles (122) représentant une cuvette est formée par un creux-empreinte de la feuille de réception (102) dans la feuille de réception (102) elle-même.

5. Dispositif de réception (100) selon l'une quelconque des revendications 1 à 4, **caractérisé en ce que** la feuille de réception (102) est en un matériau se décomposant lentement dans l'eau, en particulier en papier.

6. Dispositif de réception (100) selon l'une quelconque des revendications 1 à 5, **caractérisé en ce que** la première zone de fixation (108) et la au moins deuxième zone de fixation (110) sont formées par pulvérisation d'un adhésif par fusion d'une base ne présentant pas de risque écologique.

7. Dispositif de réception (100) selon l'une quelconque des revendications 1 à 6, **caractérisé en ce que** la première zone de fixation (108) et la au moins deuxième zone de fixation (110) sont recouvertes pour le transport et/ou dans un but de conservation d'une première contre-couche (118) et au moins d'une deuxième contre-couche (120).

8. Dispositif de réception (100) selon la revendication 7, **caractérisé en ce que** le matériau de la première contre-couche (118) et la au moins deuxième contre-couche (120) est traité pour réduire l'effet d'adhérence, en particulier par application d'une substance repoussant l'adhésif (138).

9. Dispositif de réception (100) selon la revendication 8, **caractérisé en ce que** la substance repoussant l'adhésif (138) comprend des disaccharides, en particulier du maltose et du glucose, ainsi que des polysaccharides, du lactose et des alcools à chaîne plus longue et repose sur une base aqueuse.

10. Dispositif de réception (100) selon l'une quelconque des revendications 7 à 9, **caractérisé en ce que** la première contre-couche (118) et la au moins deuxième contre-couche (120) sont formées à la feuille de réception (102) et appliquées contre la première zone de fixation (108) et la au moins deuxième zone de fixation (110) par pliage aux endroits de pliage (112, 114, 116) du matériau de la feuille de réception (102) pour le transport et/ou dans un but de conservation.

11. Dispositif de réception (100) selon la revendication 10, **caractérisé en ce que** la première zone de fixation (108) et la au moins deuxième zone de fixation (110) ne sont pas appliquées contre la première contre-couche (118) ou la au moins deuxième contre-couche (120) sur la totalité de la surface, ce pourquoi le matériau de la première et de la au moins deuxième contre-couche (118, 120) présente des zones en relief ou en creux (134, 136).

12. Dispositif de réception (100) selon l'une quelconque des revendications 1 à 11, **caractérisé en ce que** la totalité de la feuille de réception (102), à l'exception des zones de fixation (108, 110), est modifiée par empreinte de façon à ce que le matériau, dans des zones avec empreinte, présente des zones en relief ou en creux (134, 136).
